# EUROPÄISCHE PATENTSCHRIFT

(11) **EP 1 699 748 B1**
(45) Veröffentlichungstag und Bekanntmachung des Hinweises auf die Patenterteilung: **15.10.2008**
(21) Anmeldenummer: 04804198.2
(22) Anmeldetag: 22.12.2004
(51) Int. Cl.: C07C 51/09

(54) **VERFAHREN ZUR HERSTELLUNG VON SUBSTITUIERTEN 2-(PHENOXYMETHYL)-BENZOESÄUREN**
METHOD FOR THE PRODUCTION OF SUBSTITUTED 2-(PHENOXYMETHYL)-BENZOIC ACIDS
PROCEDE POUR PRODUIRE DES ACIDES 2-(PHENOXYMETHYL)-BENZOIQUES SUBSTITUES

(30) Priorität: 22.12.2003 DE 10360525
(43) Veröffentlichungstag der Anmeldung: 13.09.2006
(73) Patentinhaber: Sanofi-Aventis Deutschland GmbH, 65929 Frankfurt am Main (DE)
(72) Erfinder: FIEDLER, Wolfgang, 65926 Frankfurt am Main (DE); NEISES, Bernd, 77652 Offenburg (DE); HACHTEL, Jochen, 65929 Frankfurt (DE)
(86) Internationale Anmeldenummer: PCT/EP2004/014602
(87) Internationale Veröffentlichungsnummer: WO 2005/061427

(56) Entgegenhaltungen:
- WO-A-00/64876
- A. BASSOLI: "Quantitative Structure-Activity Relationsships of Sweet Isovanillyl Derivatives" QUANT. STRUCT.-ACT. RELAT., Bd. 20, 2001, Seiten 3-16, XP009051902
- DATABASE WPI Section Ch, Week 199511 Derwent Publications Ltd., London, GB; Class B03, AN 1995-077981 XP002339802 & JP 07 002733 A (KYOWA HAKKO KOGYO KK) 6. Januar 1995 (1995-01-06)

## Beschreibung

Die Erfindung betrifft ein Verfahren zur Herstellung von substituierten 2-(Phenoxymethyl)-benzoesäuren der allgemeinen Formel (I).

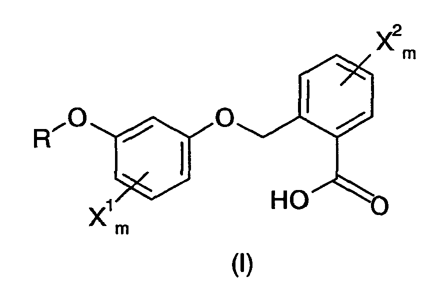

Verbindungen der allgemeinen Formel (I) eignen sich als Arzneimittel, zum Beispiel zur Senkung von Blutfetten und zur Behandlung von Diabetes, da sie eine hohe pharmakologische Wirkung als Agonist beziehungsweise Antagonist zum Peroxisomen-Proliferator-aktivierten Rezeptor (PPAR) besitzen.

Eine Vielzahl von PPAR-Agonisten beziehungsweise -Antagonisten ist bereits aus WO 00/64876 bekannt, unter anderem auch die Verbindungen der allgemeinen Formel (I).

Das in WO 00/64876 beschriebene Verfahren zur Herstellung von Verbindungen der allgemeinen Formel (I) weist allerdings einige Nachteile auf, auch im Hinblick auf eine großtechnische Herstellung dieser pharmakologisch interessanten Verbindungen. Nach dieser Herstellungsmethode wird Resorcin mit 2-Brommethyl-benzoesäureestern umgesetzt, worauf das eine freie OH-Gruppe enthaltende Zwischenprodukt mit einer Alkylierungsreagenz in Gegenwart der Base Kaliumcarbonat umgesetzt wird und abschließend durch Verseifung des Esters Verbindungen der Formel I erhalten werden. Bei diesem Verfahren ist insbesondere das Edukt 2-Brommethyl-benzoesäureester problematisch, da dieser Stoff thermisch instabil ist und schon bei Raumtemperatur unter Cyclisierung und Abspaltung von Methylbromid zum entsprechenden Phthalid zerfällt, zudem stark tränenreizend und potentiell kanzerogen und außerdem nur mittels Chromatographie rein darstellbar ist. Weiterhin ist nachteilig, dass in dem in WO 00/64876 beschriebenen Verfahren zur Herstellung des Edukts (2-Brommethyl-benzoesäureester) N-Brom-succinimid und der Explosivstoff Dibenzoylperoxid eingesetzt werden. Alternativ kann auch das Edukt durch photochemische Bromierung nach J. Org. Chem. 1985, 50, 3355 - 3359 hergestellt werden. Solche Bromierungsreaktionen zur Herstellung des Edukts sind immer dann problematisch, sofern der entsprechende Benzoesäureester über weitere Alkylgruppen als diejenige in Position 2 verfügt, da diese ebenfalls bromiert werden können. Beispielsweise weisen einige pharmakologisch interessante Verbindungen der Formel (I) ein 6-Methyl-benzoesäurefragment auf, so dass eine großtechnische Herstellung solcher Verbindungen nach dem vorgenannten Verfahren auch wegen der nicht selektiven Bromierung mit deutlich höheren Kosten infolge der zusätzlichen Aufreinigungsschritte verbunden wäre.

Alternativen zu dem in WO 00/64876 beschriebenen Verfahren für die Herstellung von 2-Carboxybenzylarylethern sind bereits bekannt. So bauen sämtliche Alternativen auf das in J. Chem. Soc. 1964, 4074 - 4075 beschriebene Verfahren auf, in dem Phenol mit dem entsprechenden Phthalid in Gegenwart der Base Natriumhydroxid bei Temperaturen über 170 °C umgesetzt wird. So betrifft US 5,221,762 ein Verfahren zur Herstellung von E-Oximethern von Phenylglyoxylsäureestern, wobei 2-Carboxybenzylarylether als Zwischenprodukt gebildet werden. Das Arylfragment kann hierbei mit C₁-C₄-Alkoxy meta-substituiertes Phenyl sein. Die Herstellung des Zwischenproduktes erfolgt in Gegenwart einer Base bei Temperaturen zwischen 50 °C und 250 °C in der Schmelze. Entsprechend wird in DE-A 2 208 893 für die Herstellung von tricyclischen α-Oxycarbonsäurederivaten von dem Edukt Guajacol ausgegangen, wobei nach Zugabe einer Methylatlösung zuerst das Lösungsmittel Methanol abdestilliert wird und nach Zugabe des Phthalids das Reaktionsgemisch auf 180 bis 190 °C erhitzt wird.

2-Carboxybenzylarylether müssen nicht zwangsweise in der Schmelze hergestellt werden, sondern es lassen sich gegebenenfalls auch höhersiedende Lösungsmittel einsetzen. In DE-A 27 49 957 ist ein Verfahren zur Herstellung von Chinolizidyliden-Derivaten von Xanthenen, Thioxanthenen und Dibenzoxepinen beschrieben, in welchem 4-Methylphenol unter Verwendung von Natriumhydrid als Base sowie Dimethylformamid als Lösungsmittel mit Phthalid unter Rückfluss umgesetzt wird. Sinngemäß wird in JP-A 07002733 die Herstellung von 2-Carboxybenzylarylethern unter Verwendung von Alkoholaten als Base sowie höhersiedenden Lösungsmitteln beschrieben. Das Arylfragment kann unter anderem auch ein mit Nieder-Alkoxy in meta-Position substituiertes Phenyl sein.

Allen vorstehend beschriebenen Verfahren zur Herstellung von 2-Carboxybenzylarylethern ist gemein, dass die Umsetzung des Alkohols mit dem Phthalid bei hohen Temperaturen (mindestens 100 °C) erfolgen muss, weil sonst keine oder nur eine geringfügige Reaktion stattfindet. Weiterhin ist festzustellen, dass in keinem Falle Resorcin beziehungsweise ein Derivat davon mit zwei freien Hydroxygruppen eingesetzt wird. Sofern die verwendeten Phenole zusätzliche Alkoxy-Substituenten (nicht nur in meta-Position) aufweisen, so sind dies unsubstituierte Nieder-Alkoxy-Substituenten. Die in WO 00/64876 beschriebenen PPAR-Agonisten beziehungsweise -Antagonisten der allgemeinen Formel (I) sind jedoch Verbindungen, die als Rest R vorzugsweise Arylalkyl- oder Heteroarylalkyl-Substituenten aufweisen, die wiederum einfach oder mehrfach substituiert sein können. Somit handelt es sich überwiegend um Reste R mit einem mittleren bis großen Molekülgewicht. Ein Verfahren analog zu beispielsweise JP-A 07002733 kann jedoch zur Herstellung von Verbindungen gemäß Formel I nicht eingesetzt werden, da aufgrund der zur Umsetzung von Alkohol und Phthalid erforderlichen hohen Temperaturen die Etherbindung zwischen R und dem Phenylfragment des Alkohols instabil ist. Je höher die Temperatur und je höher das Molekülgewicht des entsprechenden Substituenten R ist, umso geringer ist die Ausbeute an Verbindungen der allgemeinen Formel (I).

Demzufolge besteht die der Erfindung zugrundeliegende Aufgabe darin, dass ein Verfahren zur Herstellung von PPAR-Agonisten beziehungsweise -Antagonisten der allgemeinen Formel (I) bereitgestellt werden soll, das nicht die Nachteile der aus dem Stand der Technik bekannten Verfahren aufweist.

Die Aufgabe wird gelöst durch ein Verfahren zur Herstellung einer Verbindung der allgemeinen Formel (I), **dadurch gekennzeichnet, dass**
a) eine Verbindung (II) in Gegenwart einer äqumolaren Menge einer Base B1 mit einer Verbindung (III) umgesetzt wird und 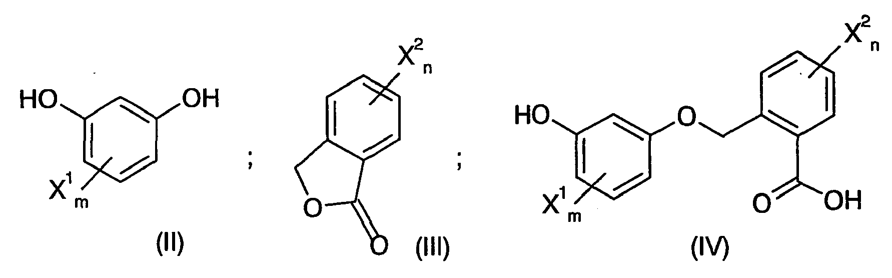
b) die in Schritt a) als Zwischenprodukt gebildete Verbindung (IV) in Gegenwart einer Base B2 mit einer Verbindung (V) zur Verbindung gemäß der allgemeinen Formel (I) umgesetzt wird, 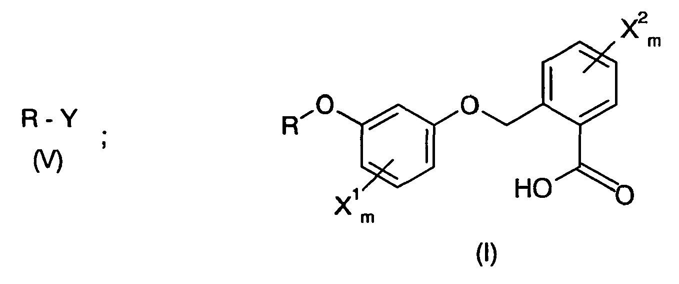 worin:
   R ist ausgewählt aus der Gruppe bestehend aus:
   unsubstituiertes oder zumindest monosubstituiertes C₁-C₁₀-Alkyl, Heterocyclyl, Aryl-(C₁-C₁₀-alkyl)-, Heteroaryl-(C₁-C₁₀-alkyl)- und Heterocyclyl-(C₁-C₁₀-alkyl)-,
wobei die Substituenten ausgewählt sind aus Halogen, C₁-C₆-Alkyl, -O-Aryl, Oxo, C₁-C₆-Alkoxy, -C(O)O-(C₁-C₆-Alkyl), C₂-C₆-Alkenyl, C₂-C₆-Alkinyl, -C(O)-(C₁-C₆-Alkyl), -C(O)NH₂, -C(O)NH(C₁-C₆-Alkyl), -C(O)N(C₁-C₆-Alkyl)₂, -S-(C₁-C₆-Alkyl), -SO₂NH₂, -SO₂-(C₁-C₆-Alkyl), -NH₂, -N(C₁-C₆-Alkyl)₂, -NH(C₁-C₆-Alkyl), -NO₂, -CN, Trifluormethyl, Trifluormethoxy, Aryl, Heterocyclyl und Heteroaryl,
und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆-Alkoxy, Trifluormethyl, Aryl, Heterocyclyl oder Heteroaryl,
wobei Aryl, Heterocyclyl und Heteroaryl wiederum mit C₁-C₃-Alkyl, C₁-C₃-Alkoxy, Halogen oder Trifluormethyl zumindest monosubstituiert sein können;
X² ist Halogen, C₁-C₆-Alkyl, C₁-C₆-Alkoxy, Trifluormethyl, Aryl, Heterocyclyl oder Heteroaryl,
wobei Aryl, Heterocyclyl und Heteroaryl wiederum mit C₁-C₃-Alkyl, C₁-C₃-Alkoxy, Halogen oder Trifluormethyl zumindest monosubstituiert sein können;
Heteroaryl ist ein 5- bis 14-gliedriger, aromatischer, mono-, bi- oder tricyclischer Heterocyclus, der ein oder mehrere Heteroatome ausgewählt aus N, O und S enthält;
Aryl ist ein 6- bis 14-gliedriger, aromatischer Mono-, Bi- oder Tricyclus;
Heterocyclyl ist ein 5- bis 14-gliedriger, nicht-aromatischer, mono-, bi- oder tricyclischer Heterocyclus, der ein oder mehrere Heteroatome ausgewählt aus N, O und S enthält;
m ist 0, 1, 2, 3 oder 4;
n ist 0,1, 2, 3 oder 4;
Y ist eine Abgangsgruppe und

B2 ist ein Alkalihydroxid, Erdalkalihydroxid, Alkalialkoholat, Erdalkalialkoholat, Alkalihydrid, Erdalkalihydrid, Silazid, Alkaliamid oder Erdalkaliamid.

Das erfindungsgemäße Verfahren hat gegenüber denjenigen aus dem Stand der Technik die Vorteile, dass einheitliche Produkte von Verbindungen gemäß der allgemeinen Formel (I) in hohen Ausbeuten und/oder mit weniger Zwischenstufen hergestellt werden können, was insbesondere hinsichtlich der großtechnischen, industriellen Nutzung ökonomisch sinnvoll ist. Gegenüber den in WO 00/64876 beschriebenen Verfahren wird zum einen auf die Verwendung von kanzerogenen und instabilen Zwischenverbindungen verzichtet, weiterhin kann aufgrund der geeigneten Basenwahl die freie Benzoesäure bei der Alkylierungsreaktion in Schritt b) eingesetzt werden. Dies ist eine Ersparnis von zwei Syntheseschritten, da bei den in WO 00/64876 beschriebenen Verfahren zuerst die freie Benzoesäure in Form eines geeigneten Esters geschützt und in der abschließenden Reaktionsstufe wieder entschützt werden muss. Der Vorteil der sinngemäßen Umkehrung der Reaktionsschritte a) und b) gegenüber den Verfahren, wie sie beispielsweise in JP-A 07002733 (Kopplung von meta-Niederalkoxyphenolen mit Phthalid) beschrieben werden, liegt darin, dass auf diese Weise Verbindungen der Formel (I) überhaupt erst oder in ökonomisch sinnvollen Ausbeuten hergestellt werden können, da die in Schritt b) des erfindungsgemäßen Verfahrens durchgeführte Alkylierungsreaktion bei deutlich niedrigeren Temperaturen als die in Schritt a) erfolgte Etherverknüpfung durchgeführt werden kann.

Mit dem erfindungsgemäßen Verfahren lassen sich Verbindungen der allgemeinen Formel (I) herstellen, 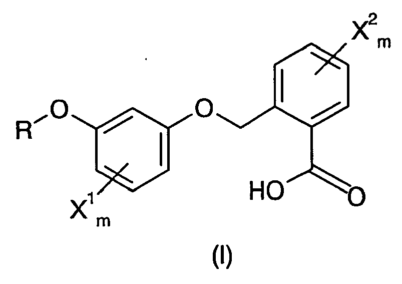 worin:
R ist ausgewählt aus der Gruppe bestehend aus:
unsubstituiertes oder zumindest monosubstituiertes C₁-C₁₀-Alkyl, Heterocyclyl, Aryl-(C₁-C₁₀-alkyl)-, Heteroaryl-(C₁-C₁₀-alkyl)- und Heterocyclyl-(C₁-C₁₀-alkyl)-,
wobei die Substituenten ausgewählt sind aus Halogen, C₁-C₆-Alkyl, -O-Aryl, Oxo, C₁-C₆-Alkoxy, C₂-C₆-Alkenyl, C₂-C₆-Alkinyl, -C(O)-(C₁-C₆-Alkyl), -C(O)NH₂, -C(O)NH(C₁-C₆-Alkyl), -C(O)N(C₁-C₆-Alkyl)₂, -S-(C₁-C₆-Alkyl), -SO₂NH₂, -SO₂-(C₁-C₆-Alkyl), -C(O)O-(C₁-C₆-Alkyl), -NH₂, -N(C₁-C₆-Alkyl)₂, -NH(C₁-C₆-Alkyl), -NO₂, -CN, Trifluormethyl, Trifluormethoxy, Aryl, Heterocyclyl und Heteroaryl,
und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆-Alkoxy, Trifluormethyl, Aryl, Heterocyclyl oder Heteroaryl,
wobei Aryl, Heterocyclyl und Heteroaryl wiederum mit C₁-C₃-Alkyl, C₁-C₃-Alkoxy, Halogen oder Trifluormethyl zumindest monosubstituiert sein können;
X² ist Halogen, C₁-C₆-Alkyl, C₁-C₆-Alkoxy, Trifluormethyl, Aryl, Heterocyclyl oder Heteroaryl,
wobei Aryl, Heterocyclyl und Heteroaryl wiederum mit C₁-C₃-Alkyl, C₁-C₃-Alkoxy, Halogen oder Trifluormethyl zumindest monosubstituiert sein können;
Heteroaryl ist ein 5- bis 14-gliedriger, aromatischer, mono-, bi- oder tricyclischer Heterocyclus, der ein oder mehrere Heteroatome ausgewählt aus N, O und S enthält;
Aryl ist ein 6- bis 14-gliedriger, aromatischer Mono-, Bi- oder Tricyclus;
Heterocyclyl ist ein 5- bis 14-gliedriger, nicht-aromatischer, mono-, bi- oder tricyclischer Heterocyclus, der ein oder mehrere Heteroatome ausgewählt aus N, O und S enthält;
m ist 0,1, 2, 3 oder 4;
n ist 0, 1, 2, 3 oder 4.

Sofern in den Verbindungen gemäß der Formel (I) Gruppen, Fragmente, Reste oder Substituenten wie beispielsweise Aryl, Heteroaryl, Alkyl, Alkoxy usw. mehr als einfach vorhanden sind, haben sie alle unabhängig voneinander die oben aufgeführten Bedeutungen und können somit in jedem (Einzel)-Fall entweder eine identische oder eine voneinander unabhängige Bedeutung haben. Die nachfolgenden Ausführungen gelten für (beispielsweise) Aryl sowie jeden beliebigen anderen Rest unabhängig von dessen Bezeichnung als Arylgruppe, -substituent, -fragment oder -rest. Zum Beispiel können in einer Di(C₁-C₆-alkyl)amino-Gruppe die beiden Alkylsubstituenten entweder identisch oder unterschiedlich sein (beispielsweise 2 x Ethyl oder 1 x Propyl und 1 x Hexyl).

Sofern in den vorstehenden Definitionen für Verbindungen gemäß Formel (I) ein Substituent, beispielsweise Aryl, unsubstituiert oder zumindest monosubstituiert mit einer Gruppe von weiteren Substituenten, beispielsweise C₁-C₆-Alkyl, C₁-C₆-Alkoxy, Halogen usw., sein kann, so gilt für diejenigen Fälle, in denen eine Mehrfachsubstitution von Aryl vorliegt, dass die Auswahl aus der Reihe von weiteren Substituenten unabhängig voneinander erfolgt. Somit sind beispielsweise bei einer Zweifachsubstitution von Aryl sämtliche Kombinationen der weiteren Substituenten mit umfasst. Aryl kann somit beispielsweise zweifach-substituiert mit Ethyl sein, Aryl kann jeweils monosubstituiert mit Methyl und Ethoxy sein, Aryl kann jeweils monosubstituiert mit Ethyl und Fluor sein, Aryl kann zweifach-substituiert mit Methoxy sein, usw..

Alkylreste können entweder linear oder verzweigt, acyclisch oder cyclisch sein. Dies trifft auch zu, sofern sie Teil einer anderen Gruppe wie beispielsweise Alkoxygruppen (C₁-C₁₀-Alkyl-O-), Alkoxycarbonylgruppen oder Aminogruppen sind oder wenn sie substituiert sind.

Beispiele für Alkylgruppen sind: Methyl, Ethyl, Propyl, Butyl, Pentyl, Hexyl, Heptyl, Octyl, Nonyl oder Decyl. Dabei sind sowohl die n-Isomere dieser Reste als auch Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise 1, 2, 3 oder 4 identische oder unterschiedliche Reste, wie beispielsweise Aryl, Heteroaryl, Alkoxy oder Halogen. Dabei können die zusätzlichen Substituenten in jeder beliebigen Position des Alkylrestes auftreten. Weiterhin umfasst der Begriff Alkyl auch Cycloalkyl sowie Cycloalkyl-alkyl- (Alkyl, das wiederum mit Cycloalkyl substituiert ist), wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl. Der Begriff Alkenyl umfasst hier ausdrücklich auch Cycloalkenylreste sowie Cycloalkenyl-Alkylreste (Alkyl, das mit Cycloalkenyl substituiert ist), die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen, für Alkenylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt.

Sofern nicht anders ausgeführt, können die vorgenannten Aryl-, Heteroaryl- und Heterocyclylreste sowohl unsubstituiert sein als auch einen oder mehrere, beispielsweise 1, 2, 3 oder 4 weitere, der vorgenannten Substituenten in jeder beliebigen Position aufweisen. Beispielsweise kann in monosubstituierten Phenylresten der Substituent in der Position 2, 3 oder 4, in disubstituierten Phenylresten können die Substituenten in der 2,3-Position, 2,4-Position, 2,5-Position, 2,6-Position, 3,4-Position oder 3,5-Position sein. In dreifach substituierten Phenylresten können die Substituenten in der 2,3,4-Position, 2,3,5-Position, 2,3,6-Position, 2,4,5-Position, 2,4,6-Position oder 3,4,5-Position sein. In vierfach substituierten Phenylresten können die Substituenten in der 2,3,4,5-Position, der 2,3,4,6-Position oder in der 2,3,5,6-Position sein.

Die vorgenannten beziehungsweise die nachfolgenden Definitionen, die sich auf einwertige Reste beziehen, gelten genauso für zweiwertige Reste wie Phenylen, Naphthylen oder Heteroarylen. Diese zweiwertigen Reste (Fragmente) können mit dem benachbarten Gruppen über jedes beliebige Ring-Kohlenstoffatom verknüpft sein. Im Falle von Phenylenresten kann dies in der 1,2-Position (Ortho-Phenylen), 1,3-Position (meta-Phenylen) oder 1,4-Position (Para-Phenylen) sein. Im Falle eines 5-gliedrigen Aromaten, der ein Heteroatom enthält, wie beispielsweise Thiophen oder Furan können die beiden freien Bindungen in der 2,3-Position, 2,4-Position, 2,5-Position oder der 3,4-Position sein. Ein zweiwertiger Rest, der von einem 6-gliedrigen Aromaten mit einem Heteroatom abgeleitet ist, wie beispielsweise Pyridin, kann ein 2,3-, 2,4-, 2,5-, 2,6-, 3,4- oder 3,5-Pyridindiylrest sein. Im Falle von unsymmetrischen zweiwertigen Resten beinhaltet die vorliegende Erfindung auch alle Positionsisomere, d.h. im Falle von beispielsweise einem 2,3-Pyridindiylrest ist die Verbindung, in der die eine benachbarte Gruppe sich in der Position 2 und die andere benachbarte Gruppe in der Position 3 sich befindet, genauso wie die Verbindung, in der die eine benachbarte Gruppe in der Position 3 und die andere benachbarte Gruppe sich in der Position 2 befindet, mit umfasst.

Soweit nicht anders aufgeführt, sind Heteroarylreste, Heteroarylenreste, Heterocyclylreste und Heterocyclylenreste sowie Ringe, die durch zwei an Stickstoff gebundene Gruppen gebildet werden, vorzugsweise abgeleitet von vollständig gesättigten, teilweise oder ganz ungesättigten Heterocyclen (d.h. Heterocycloalkane, Heterocycloalkene, Heteroaromaten), die 1, 2, 3 oder 4 Heteroatome enthalten, die sowohl unterschiedlich als auch gleich sein können. Vorzugsweise sind sie von Heterocyclen abgeleitet, die 1, 2 oder 3, besonders bevorzugt 1 oder 2, Heteroatome enthalten, die gleich oder unterschiedlich sein können. So lange nicht anders aufgeführt, sind die Heterocyclen mono- oder polycyclisch, zum Beispiel monocyclisch, bicyclisch oder tricyclisch. Vorzugsweise sind sie monocyclisch oder bicyclisch. Vorzugsweise sind es 5-gliedrige, 6-gliedrige oder 7-gliedrige Ringe, besonders bevorzugt 5-gliedriger oder 6-gliedrige Ringe. Im Fall von polycyclischen Heterocyclen mit 2 und mehr Heteroatomen können diese alle im gleichen Ring vorkommen oder auf mehrere Ringe verteilt sein.

Als Heteroaryl werden in der vorliegenden Erfindung Reste bezeichnet, die von monocyclischen, bicyclischen oder tricyclischen, aromatischen Heterocyclen abgeleitet sind. Beispiele für Heteroaryl sind: Pyrrolyl, Furanyl (Furyl), Thiophenyl (Thienyl), Imidazolyl, Pyrazolyl, 1,2,3-Triazolyl, 1,2,4-Triazolyl, 1,3-Oxazolyl (Oxazolyl), 1,2-Oxazolyl (Isoxazolyl), Oxadiazolyl, 1,3-Thiazolyl (Thiazolyl), 1,2-Thiazolyl (Isothiazolyl), Tetrazolyl, Pyridinyl (Pyridyl), Pyridazinyl, Pyrimidinyl, Pyrazinyl, 1,2,3-Triazinyl, 1,2,4-Triazinyl, 1,3,5-Triazinyl, 1,2,4,5-Tetrazinyl, Indazolyl, Indolyl, Benzothiophenyl, Benzofuranyl, Benzothiazolyl, Benzimidazolyl, Benzodioxolyl, Acridinyl, Chinolinyl, Isochinolinyl, Chinazolinyl, Chinoxalinyl, Phthalazinyl, Thienothiophenyl, 1,8-Naphthyridinyl, andere Naphthyridinyle, Pteridinyl oder Phenothiazinyl. Sofern es sich nicht um monocyclische Systeme handelt, ist bei jedem der vorgenannten Heteroaryle für jeden zusätzlichen Ring auch die gesättigte Form (Perhydroform) oder die teilweise ungesättigte Form (beispielsweise die Dihydroform oder Tetrahydroform) oder die maximal ungesättigte (nicht-aromatische) Form mitumfasst, sofern die jeweiligen Formen bekannt und stabil sind. Die Bezeichnung Heteroaryl umfasst somit in der vorliegenden Erfindung beispielsweise auch bicyclische Reste, in denen sowohl beide Ringe aromatisch sind als auch bicyclische Reste, in denen nur ein Ring aromatisch ist. Solche Beispiele für Heteroaryl sind: 3H-Indolinyl, 2(1H)-Chinolinonyl, 4-Oxo-1,4-dihydrochinolinyl, 2H-1-Oxoisochinolyl, 1,2-Dihydrochinolinyl, (2H)Chinolinyl-N-oxid, 3,4-Dihydrochinolinyl, 1,2-Dihydroisochinolinyl, 3,4-Dihydroisochinolinyl, Chromonyl, 3,4-Dihydroisochinoxalinyl, 4-(3H)Chinazolinonyl, 4H-Chromenyl, 4-Chromanonyl, Oxindolyl, 1,2,3,4-Tetrahydroisochinolinyl, 1,2,3,4- Tetrahydrochinolinyl, 1H-2,3-Dihydroisoindolyl, 2,3-Dihydrobenz[f]isoindolyl, 1,2,3,4-Tetrahydrobenz[g]isochinolinyl, Chromanyl, Isochromanonyl, 2,3-Dihydrochromonyl, 1,4-Benzodioxanyl, 1,2,3,4-Tetrahydrochinoxalinyl, 5,6-Dihydrochinolyl, 5,6-Dihydroisochinolyl, 5,6-Dihydrochinoxalinyl, 5,6-Dihydrochinazolinyl, 4,5-Dihydro-1H-benzimidazolyl, 4,5-Dihydrobenzoxazolyl, 1,4-Naphthochinolyl, 5,6,7,8-Tetrahydrochinolinyl, 5,6,7,8-Tetrahydroisochinolyl, 5,6,7,8-Tetrahydrochinoxalinyl, 5,6,7,8-Tetrahydrochinazolyl, 4,5,6,7-Tetrahydro-1H-benzimidazolyl, 4,5,6,7-Tetrahydrobenzoxazolyl, 1H-4-Oxa-1,5-diazanaph-thalen-2-onyl, 1,3-Dihydroimidizole-[4,5]-pyridin-2-onyl, 2,3-Dihydro-1,4-dinaphtho-chinonyl, 2,3-Dihydro-1Hpyrrol[3,4-b]chinolinyl, 1,2,3,4-Tetrahydrobenz[b][1,7]naphthyridinyl, 1,2,3,4-Tetrahydrobenz[b] [1,6]naphthyridinyl, 1,2,3,4-Tetrahydro-9H-pyrido[3,4-b]indolyl, 1,2,3,4-Tetrahydro-9H-pyrido[4,3-b]indolyl, 2,3-Dihydro-1H-pyrrolo[3,4-b]indolyl, 1H-2,3,4,5-Tetrahydroazepino[3,4-b]indolyl, 1H-2,3,4,5-Tetrahydroazepino[4,3-b]indolyl, 1H-2,3,4,5-Tetrahydroazepino[4,5-b]indolyl, 5,6,7,8-Tetrahydro[1,7]napthyridinyl, 1,2,3,4-Tetrahydro[2,7]naphthyridyl, 2,3-Dihydro[1,4]dioxino[2,3-b]pyridyl, 2,3-Dihydro[1,4]dioxino[2,3-b]pryidyl, 3,4-Dihydro-2H-1-oxa[4,6]diazanaphthalenyl, 4,5,6,7-Tetrahydro-3H-imidazo[4,5-c]pyridyl, 6,7-Dihydro[5,8]diazanaphthalenyl, 1,2,3,4-Tetrahydro[1,5]napthyridinyl, 1,2,3,4-Tetrahydro[1,6]napthyridinyl, 1,2,3,4-Tetrahydro[1,7]napthyridinyl, 1,2,3,4-Tetrahydro[1,8]napthyridinyl oder 1,23,4-Tetrahydro[2,6]napthyridinyl.

Als Heterocyclyl werden in der vorliegenden Erfindung Reste bezeichnet, die von monocyclischen, bicyclischen oder tricyclischen, nicht-aromatischen Heterocyclen abgeleitet sind. Unter nicht-aromatischen Heterocyclen werden nachfolgend insbesondere Heterocycloalkane (vollständig gesättigte Heterocyclen) sowie Heterocycloalkene (teilweise ungesättigte Heterocyclen) verstanden. Im Fall der Heterocycloalkene werden auch Verbindungen mit zwei oder mehreren Doppelbindungen mit umfasst, die gegebenenfalls auch miteinander konjugiert sein können. Beispiele für Heterocyclyl sind: Pyrrolidinyl, Piperidinyl, Piperazinyl, Imidazolidinyl, Pyrazolidinyl, Isothiazolidinyl, Thiazolidinyl, Isoxazolidinyl, Oxazolidinyl, Tetrahydrofuranyl, Tetrahydrothiophenyl, 1,3-Dioxolanyl, Pyranyl, Thiopyranyl, 1,4-Dioxinyl, 1,2-Oxazinyl, 1,3-Oxazinyl, 1,4-Oxazinyl, 1,2-Thiazinyl, 1,3-Thiazinyl, 1,4-Thiazinyl, Azepinyl, 1,2-Diazepinyl, 1,3-Diazepinyl, 1,4-Diazepinyl, 1,3-Oxazepinyl, 1,3-Thiazepinyl, 2-Oxo-azepanyl, Morpholinyl, Thiomorpholinyl, 1,2,3,4- Tetrahydropyridinyl, 1,2-Dihydropyridinyl, 1,4-Dihydropyridinyl, 1,2,3,6-Tetrahydropyridinyl, 4(3H)-pyrimidonyl, 1,4,5,6-Tetrahydropyrimidinyl, 2-Pyrrolinyl, 3-Pyrrolinyl, 2-Imidazolinyl, 2-Pyrazolinyl, 3,4-Dihydro-2H-pyranyl, Dihydrofuranyl, 7-Oxabicyclo[2.2.1]heptenyl, Dihydrothiophenyl und Dihydrothiopyranyl. Der Grad der Sättigung von heterocyclischen Gruppen ist in der jeweiligen Definition angezeigt.

Substituenten, die von diesen Heterocyclen abgeleitet sind, können über jedes geeignete Kohlenstoffatom verknüpft sowie mit weiteren Substituenten versehen sein. Reste, die von stickstoffhaltigen Heterocyclen abgeleitet sind, können am entsprechenden Stickstoffatom ein Wasserstoffatom oder einen anderen Substituenten aufweisen. Beispiele umschließen Pyrrol, Imidazol, Pyrrolidin, Morpholin, Piperazinreste usw.. Diese stickstoffhaltigen heterocyclischen Reste können auch über das Ring-Stickstoffatom gebunden sein, insbesondere wenn der entsprechende heterocyclische Rest an ein Kohlenstoffatom gebunden ist. Beispielsweise kann ein Thienylrest als 2-Thienyl oder 3-Thienyl vorliegen, ein Piperidinylrest als 1-Piperidinyl (Piperidino), 2-Piperidinyl, 3-Piperidinyl oder 4-Piperidinyl. Geeignete stickstoffhaltige Heterocyclen können auch als N-Oxide oder als quarternäre Salze, die ein Gegenion aufweisen, das von einer physiologisch annehmbaren Säure abgeleitet ist, vorliegen. Beispielsweise können Pyridylreste als Pyridin-N-oxide vorliegen. Geeignete schwefelhaltige Heterocyclen können auch als S-Oxid oder S,S-Dioxid vorliegen.

Als Aryl werden in der vorliegenden Erfindung Reste bezeichnet, die von monocyclischen, bicyclischen oder tricyclischen Aromaten abgeleitet sind, die keine Ringheteroatome enthalten. Sofern es sich nicht um monocyclische Systeme handelt, ist bei der Bezeichnung Aryl für jeden zusätzlichen Ring auch die gesättigte Form (Perhydroform) oder die teilweise ungesättigte Form (beispielsweise die Dihydroform oder Tetrahydroform) oder die maximal ungesättigte (nicht-aromatische) Form mit umfasst, sofern die jeweiligen Formen bekannt und stabil sind. Die Bezeichnung Aryl umfasst in der vorliegenden Erfindung auch beispielsweise bicyclische Reste, in denen sowohl beide Ringe aromatisch sind als auch bicyclische Reste, in denen nur ein Ring aromatisch ist. Beispiele für Aryl sind: Phenyl, Naphthyl Anthracyl, Indanyl, 1,2-Dihydronaphthyl, 1,4-Dihydronaphthyl, Indenyl, 1,4-Naphthochinonyl oder 1,2,3,4-Tetrahydronaphthyl.

Arylalkyl bedeutet, dass ein Alkylrest wiederum mit einem Arylrest substituiert ist. Heteroarylalkyl bedeutet, dass ein Alkylrest wiederum mit einem Heteroarylrest substituiert ist. Heterocyclylalkyl bedeutet, dass ein Alkylrest wiederum mit einem Heterocyclylrest substituiert ist. Für die Definitionen und Substitutionsmöglichkeiten von Alkyl, Heteroaryl, Heterocyclyl und Aryl wird auf die vorstehenden Definitionen verwiesen.

Halogen ist Fluor, Chlor, Brom oder Iod, bevorzugt ist Fluor, Chlor, oder Brom, besonders bevorzugt ist Fluor oder Chlor.

Bevorzugte Verbindungen der allgemeinen Formel (I), die mit dem erfindungsgemäßen Verfahren hergestellt werden können, sind wie folgt definiert:
R ist ausgewählt aus der Gruppe bestehend aus:
   unsubstituiertes oder zumindest monosubstituiertes C₁-C₁₀-Alkyl, Heterocyclyl, Aryl-(C₁-C₁₀-alkyl)-, Heteroaryl-(C₁-C₁₀-alkyl)- und Heterocyclyl-(C₁-C₁₀-alkyl)-,
   wobei die Substituenten ausgewählt sind aus Halogen, C₁-C₆-Alkyl, -O-Aryl, Oxo, C₁-C₆-Alkoxy, -C(O)O-(C₁-C₆-Alkyl), C₂-C₆-Alkenyl, C₂-C₆-Alkinyl, -C(O)-(C₁-C₆-Alkyl), -C(O)NH₂, -C(O)NH(C₁-C₆-Alkyl), -C(O)N(C₁-C₆-Alkyl)₂, -S-(C₁-C₆-Alkyl), -SO₂NH₂, -SO₂-(C₁-C₆-Alkyl), -NH₂, -N(C₁-C₆-Alkyl)₂, -NH(C₁-C₆-Alkyl), -NO₂, -CN, Trifluormethyl, Trifluormethoxy, Aryl, Heterocyclyl und Heteroaryl,
   und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆-Alkoxy oder Trifluormethoxy;
X² ist Halogen, C₁-C₆-Alkyl, C₁-C₆-Alkoxy oder Trifluormethoxy;
Heteroaryl ist ein 5- bis 14-gliedriger, aromatischer, mono-, bi- oder tricyclischer Heterocyclus, der ein oder mehrere Heteroatome ausgewählt aus N, O und S enthält;
Aryl ist ein 6- bis 14-gliedriger, aromatischer Mono-, Bi- oder Tricyclus;
Heterocyclyl ist ein 5- bis 14-gliedriger, nicht-aromatischer, mono-, bi- oder tricyclischer Heterocyclus, der ein oder mehrere Heteroatome ausgewählt aus N, O und S enthält;
m ist 0, 1oder 2;
n ist 0, 1 oder 2.

Mehr bevorzugte Verbindungen der allgemeinen Formel (I) sind wie folgt definiert:
R ist ausgewählt aus der Gruppe bestehend aus:
   unsubstituiertes oder zumindest monosubstituiertes Aryl-(C₁-C₆-alkyl)- und Heteroaryl-(C₁-C₆-alkyl)-,
   wobei die Substituenten ausgewählt sind aus Halogen, C₁-C₄-Alkyl, -O-Aryl, Oxo, C₁-C₄-Alkoxy, Aryl, Heterocyclyl und Heteroaryl,
   und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆-Alkoxy oder Trifluormethoxy;
X² ist Halogen, C₁-C₆-Alkyl, C₁-C₆-Alkoxy oder Trifluormethoxy;
Heteroaryl ist ein 5- bis 14-gliedriger, aromatischer, mono-, bi- oder tricyclischer Heterocyclus, der ein oder mehrere Heteroatome ausgewählt aus N, O und S enthält;
Aryl ist ein 6- bis 14-gliedriger, aromatischer Mono-, Bi- oder Tricyclus;
Heterocyclyl ist ein 5- bis 14-gliedriger, nicht-aromatischer, mono-, bi- oder tricyclischer Heterocyclus, der ein oder mehrere Heteroatome ausgewählt aus N, O und S enthält;
m ist 0, 1oder 2;
n ist 0, 1 oder 2.

Noch mehr bevorzugte Verbindungen der allgemeinen Formel (I) sind wie folgt definiert:
R ist ausgewählt aus der Gruppe bestehend aus:
   unsubstituiertes oder zumindest monosubstituiertes Aryl-(C₁-C₆-alkyl)- und Heteroaryl-(C₁-C₆-alkyl)-,
   wobei die Substituenten ausgewählt sind aus Halogen, C₁-C₄-Alkyl, -O-Aryl, Oxo, C₁-C₄-Alkoxy, Aryl, Heterocyclyl und Heteroaryl,
   und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆-Alkoxy oder Trifluormethoxy;
X² ist Halogen, C₁-C₆-Alkyl, C₁-C₆-Alkoxy oder Trifluormethoxy;
Heteroaryl ist ein 5- bis 10-gliedriger, aromatischer, mono- oder bicyclischer Heterocyclus, der ein oder mehrere Heteroatome ausgewählt aus N, O und S enthält;
Aryl ist Phenyl, Naphthyl, Indanyl, Dihydronaphthyl, Tetrahydronaphthyl oder Indenyl;
Heterocyclyl ist Pyrrolidinyl, Piperidinyl, Piperazinyl, Tetrahydrofuranyl oder Morpholinyl;
m ist 0, 1oder 2;
n ist 0,1 oder 2;

Besonders bevorzugte Verbindungen der allgemeinen Formel (I) sind wie folgt definiert:
R ist unsubstituiertes oder zumindest monosubstituiertes Benzyl oder Heteroarylmethyl, wobei die Substituenten ausgewählt sind aus Fluor, Chlor, C₁-C₄-Alkyl, -O-Phenyl, C₁-C₄-Alkoxy, Phenyl und Heteroaryl,
und Phenyl und Heteroaryl wiederum mit C₁-C₃-Alkyl, C₁-C₃-Alkoxy, Chlor oder Fluor zumindest monosubstituiert sein können;
X¹ ist C₁-C₃-Alkyl oder Brom;
X² ist C₁-C₃-Alkyl, Fluor oder Chlor;
Heteroaryl ist Oxazolyl, Isoxazolyl, Oxadiazolyl, Thiazolyl, Isothiazolyl, Pyridinyl, Pyridazinyl, Pyrimidinyl, Pyrazinyl, Indolyl, Benzimidazolyl, Chinolinyl, Isochinolinyl, Chinazolinyl oder Chinoxalinyl;
m ist 0 oder 1;
n ist 0,1 oder 2.

Ganz besonders bevorzugte Verbindungen der allgemeinen Formel (I) sind wie folgt definiert:
R ist unsubstituiertes oder zumindest monosubstituiertes Benzyl oder Heteroarylmethyl, wobei die Substituenten ausgewählt sind aus Fluor, Chlor, C₁-C₄-Alkyl, Phenyl und Heteroaryl,
   und Phenyl und Heteroaryl wiederum mit C₁-C₃-Alkyl, Chlor oder Fluor zumindest monosubstituiert sein können;
X² ist C₁-C₃-Alkyl;
Heteroaryl ist Oxazolyl oder Isoxazolyl;
m ist 0;
n ist 0 oder 1.

### Schritt a) des erfindungsgemäßen Verfahrens:

In Schritt a) des erfindungsgemäßen Verfahrens wird eine Verbindung (II) in Gegenwart einer Base B1 mit einer Verbindung (III) umgesetzt wird, wobei eine Verbindung (IV) gebildet wird.

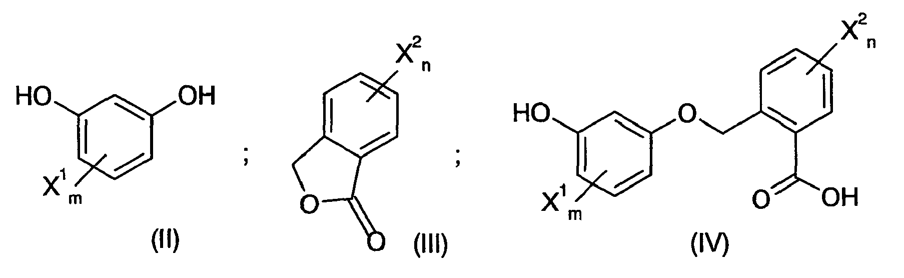

Die Verbindungen der Formeln (II) und (III) weisen die entsprechenden Definitionen der allgemeinen Formel (I) auf. Als Verbindungen (II), die nachfolgend als Resorcinderivate bezeichnet werden, eignen sich nur Verbindungen, die zwei zueinander in meta-Position stehende Hydroxylgruppen aufweisen. Die entsprechenden Hydrochinon- und Brenzcatechinderivate können im erfindungsgemäßen Verfahren jedoch nicht verwendet werden, da sie sich nicht oder nur in geringem Maße mit den Verbindungen der Formel (III), die nachfolgend als Phthalide bezeichnet werden, umsetzen lassen.

Als Basen B1 sind prinzipiell alle dem Fachmann geläufigen Basen geeignet. Es eignen sich bevorzugt Alkali- und Erdalkalialkoholate wie Natriummethylat, Kaliummethylat und Kalium tert.-butylat. Ebenso geeignet sind Silazide wie Kalium-hexamethyldisilazid und Lithium-hexamethyldisilazid. Mit Alkalihydroxiden wie Natriumhydroxid und Kaliumhydroxid lassen sich die Verbindungen der Formel (IV) ebenfalls darstellen, allerdings reagiert das bei der Deprotonierung entstehende Wasser ebenfalls mit dem eingesetzten Phthalid unter bei den Reaktionsbedingungen irreversibler Ringöffnung und entzieht damit das Edukt der gewünschten Reaktion, was Ausbeuteverluste zur Folge hat. Besonders bevorzugt als Base B1 sind Alkalialkoholate, ganz besonders bevorzugt Natriummethylat, Kaliummethylat und Kalium tert.-butylat.

Als Lösungsmittel eignen sich Lösungsmittel beziehungsweise deren Gemische mit einem Siedepunkt von > 100 °C, wie zum Beispiel Toluol, o-, m-, p-Xylol, Glyme, Dimethylformamid, N-Methyl-1-pyrrolidon und N,N-Dimethylacetamid. Besonders bevorzugt sind N-Methyl-1-pyrrolidon und N,N-Dimethylacetamid.

Die Menge an einzusetzender Base B1 ist äquimolar in Bezug auf die Menge an eingesetztem Resorcinderivat (II), bevorzugt ist die exakt äquimolare Menge.

Die Menge an eingesetzter Base B1 und Resorcinderivat (II) muss mindestens äquimolar in Bezug auf das eingesetzte Phthalid (III) sein. Die Reaktionsgeschwindigkeit kann durch gleichzeitige Verwendung größerer Mengen an Resorcinderivat (II) und an Base B1 gesteigert werden. Bevorzugt verwendet man einen ein- bis sechsmolaren Überschuss an Base B1 und Resorcinderivat (II), bezogen auf die Menge an eingesetztem Phthalid (III).

Phthalid (III), Resorcinderivat (II), Base B1 und Lösungsmittel werden bei Raumtemperatur gemischt, wobei die Reihenfolge der Zugabe keine Rolle spielt und keinen Einfluss auf den Umsatz der Reaktion und die Ausbeute des isolierten Produkts hat. Demzufolge ist es nicht nötig, wie in einigen der vorstehenden Dokumente beschrieben, zuerst die Deprotonierung des Resorcinderivats (II) mittels der verwendeten Base B1 durchzuführen, eventuell noch die korrespondierende Säure der eingesetzten Base B1 zu entfernen und anschließend das Phthalid (III) zuzugeben und zu erhitzen.

Im Allgemeinen führt man die Reaktion nach der Mischung der Reaktanden und Lösungsmittel bei einer Temperatur in einem Bereich von 80 bis 200 °C, vorzugsweise zwischen 110 und 150 °C durch. Die Reaktionszeit ist abhängig vom eingesetzten Phthalid (III) und dessen sterischer Beanspruchung.

Es sind keine besonderen Bedingungen bezüglich des Druckes erforderlich, zweckmäßigerweise arbeitet man bei Normaldruck.

Nach Beendigung der Reaktion verdünnt man das Reaktionsgemisch mit Wasser. Die Zugabe des Wassers erfolgt vorzugsweise bei Temperaturen > 60 °C, da je nach Konzentration der Reaktionsmischung ein Erstarren derselben nicht zu verhindern ist. Die erhaltene Lösung wird zur Freisetzung der Verbindungen (IV) angesäuert, bevorzugt mit einer anorganischen Säure wie Salzsäure oder Schwefelsäure. Die weitere Aufarbeitung erfolgt mit dem Fachmann bekannten Methoden.

Die bisher in der Literatur nicht bekannten Zwischenpodukte der Formel (IV) sind nach dem erfindungsgemäßen Verfahren in hohen Ausbeuten und ausgezeichneten Reinheiten zugänglich. Bei Verwendung von N-Methyl-1-pyrrolidon und N,N-Dimethylacetamid ist es möglich, dass primär Solvate der Verbindungen der Formel (IV) mit wechselnden Gehalten der genannten Lösungsmittel erhalten werden. Durch geeignete Verfahren, z.B. Umkristallisation oder Erhitzen mit Wasser lässt sich der Gehalt der genannten Lösungsmittel im isolierten Produkt reduzieren bzw. gänzlich entfernen. Zur weiteren Verwendung der Zwischenprodukte (IV) in Schritt b) des erfindungsgemäßen Verfahrens ist eine Entfernung der Solvathülle nicht erforderlich.

Das erfindungsgemäße Verfahren in Schritt a) und/oder Schritt b) kann sowohl diskontinuierlich als auch kontinuierlich durchgeführt werden. Bei einer kontinuierlichen Arbeitsweise leitet man die Reaktionspartner beispielsweise durch einen Rohrreaktor oder über Rührkesselkaskaden.

### Schritt b) des erfindungsgemäßen Verfahrens:

In Schritt b) wird die in Schritt a) des erfindungsgemäßen Verfahrens als Zwischenprodukt gebildete Verbindung (IV) in Gegenwart einer Base B2 mit einer Verbindung R-Y (V) zur Verbindung gemäß der allgemeinen Formel (I) umgesetzt.

Die in Schritt a) als Zwischenprodukt gebildete Verbindung (IV) sowie die Verbindung (V) weisen die entsprechenden Definitionen der allgemeinen Formel (I) auf. Als Abgangsgruppe Y in oder Verbindung (V) eignet sich jede dem Fachmann bekannte Abgangsgruppe. Bevorzugt sind für Y Chlor, Brom, Iod, Mesylate oder Tosylate, besonders bevorzugt Chlor, Brom oder Iod.

Als Basen B2 eignen sich Alkali- und Erdalkalihydroxide wie Natrium- und Kaliumhydroxid, Alkalimetall- und Erdalkalimetallalkoholate wie Natriummethylat, Kaliummethylat und Kalium tert.-butylat, Alkali- und Erdalkalihydride wie Natrium- und Calciumhydrid. Ebenso geeignet sich Silazide wie Kalium-hexamethyldisilazid und Lithium-hexamethyldisilazid sowie allgemein Alkali- und Erdalkaliamide; Alkalialkoholate sind bevorzugt und besonders bevorzugt sind Natriumalkoholate. Unter Verwendung der vorgenannten Basen B2 kann in Schritt b) des erfindungsgemäßen Verfahrens eine selektive Alkylierung der Hydroxylgruppe der in Schritt a) als Zwischenprodukt gebildeten Verbindung (IV) erzielt werden. Eine zusätzliche Alkylierung der ungeschützten Carboxylgruppe der Verbindung (IV) findet nicht oder nur in geringem Maße statt.

Als ungeeignete Basen haben sich carbonatische Basen erwiesen, wie zum Beispiel Kaliumcarbonat. Aufgrund der unzureichenden Basizität wird auch bei großen Überschüssen nur die Carboxylgruppe deprotoniert, was zur selektiven, aber ungewünschten Alkylierung der Säurefunktion der Verbindungen der Formel I führt. Diese selektive Alkylierung der Säurefunktion wird bei niedrigeren Temperaturen, insbesondere bei Temperaturen kleiner oder gleich 50°C, beobachtet; vermutlich aufgrund des gleichzeitigen Vorliegens einer Hydroxyl- und einer Carboxylgruppe ist die Basizität von carbonatischen Basen bei niedrigen Temperaturen nicht ausreichend.

Als Lösungsmittel eignen sich alle Lösungsmittel beziehungsweise deren Gemische, die mit den eingesetzten Basen nicht reagieren können:
i) aprotisch polare Lösungsmittel wie Aceton und Carbonsäureamide, vorzugsweise N-Methyl-1-pyrrolidinon, N,N-Dimethyl-acetamid und Dimethylformamid,
ii) protisch polare Lösungsmittel wie zum Beispiel Alkohole wie Methanol, Ethanol und tert.-Butanol.

Bevorzugt sind Alkohole und Carbonsäureamide. Besonders bevorzugt sind Carbonsäureamide.

Die Menge an eingesetztem Alkylierungsreagenz R-Y der Formel (V) liegt im Bereich von 1,0 bis 1,5 Moläquivalenten bezogen auf die eingesetzte Verbindung (IV), bevorzugt zwischen 1,0 und 1,25 Moläquivalenten. Gegebenenfalls können auch mehr als 1,5 Moläquivalente von R-Y eingesetzt werden. Die Menge an eingesetzter Base B2 beträgt mindestens die zweifach molare Menge an eingesetzter Verbindung (IV), bevorzugt ist die exakt zweifach molare Menge von Base B2 gegenüber der Verbindung (IV).

Die Reaktion wird in Abhängigkeit vom verwendeten Lösungsmittel bei Temperaturen im Bereich von 20 bis zu 60 °C, vorzugsweise von 20 bis zu 50 °C, besonders bevorzugt zwischen 20 und 25 °C, durchgeführt. Carbonsäureamide als Lösungsmittel erfordern im Allgemeinen geringere Temperaturen als Alkohole als Lösungsmittel. Gegebenenfalls kann Schritt b) auch bei Temperaturen über 60 °C durchgeführt werden.

Man legt die Verbindung (IV) im Lösungsmittel vor und gibt anschließend die Base B1 hinzu. Man lässt bis zur vollständigen Deprotonierung der beiden Hydroxylgruppen mindestens fünf Minuten rühren. Anschließend gibt man das Alkylierungsreagenz R-Y der Formel (V) hinzu.

Es sind keine besonderen Bedingungen bezüglich des Druckes erforderlich; zweckmäßigerweise arbeitet man bei Normaldruck.

Nach Beendigung der Reaktion verdünnt man das Reaktionsgemisch beispielsweise mit einer Kochsalz-, Kaliumchlorid- oder Natriumhydrogencarbonatlösung. Die erhaltene Lösung wird beispielsweise mit Ethylacetat ausgeschüttelt, wobei die organischen Bestandteile der Reaktionslösung in die organische Phase übergehen. Die EthylacetatPhase wird danach mit Wasser extrahiert. Zur Freisetzung der Verbindungen der allgemeinen Formel (I) wird die wässrige Phase angesäuert, bevorzugt mit einer anorganischen Säure wie Salzsäure oder Schwefelsäure. Die weitere Aufarbeitung erfolgt nach dem Fachmann bekannten Methoden. Man erhält die erfindungsgemäßen Verbindungen der Formel (I) in hohen Ausbeuten und guten Reinheiten.

Die Reinheit der isolierten Verbindungen der allgemeinen Formel (I) kann gegebenenfalls durch eine nachfolgende Kristallisation gesteigert werden.

In einer weiteren Ausführungsform der vorliegenden Verbindungen können im Anschluss an Schritt b) mit dem Fachmann bekannten Methoden - sofern für X¹, X² oder R definiert - aus (-C(O)O-(C₁-C₆-Alkyl))-, (-S-(C₁-C₆-Alkyl))- und/oder (C₁-C₆-Alkoxy)-Substituenten durch Alkylabspaltung die entsprechenden Carboxyl, -SH und/oder -OH substituierten Verbindungen der allgemeinen Formel (I) hergestellt werden. Diese zusätzlichen Substituenten können unabhängig voneinander einfach oder mehrfach vorhanden sein. Vorzugsweise werden die freien Carboxyl-, (-SH)- und/oder (-OH)-Substituenten durch Zugabe von Säure gewonnen. Gegebenenfalls können diese zusätzlichen Substituenten auch aus anderen Substituenten als Ester, Thioalkoxy oder Alkoxy-Substituenten gewonnen werden.

Die im erfindungsgemäßen Verfahren verwendeten Ausgangsmaterialien, Lösungsmittel, Basen usw. sind alle entweder käuflich erhältlich oder sie lassen sich nach dem Fachmann bekannten Methoden herstellen.

Eine zu den Verbindungen der allgemeinen Formel (IV) ähnliche Verbindung ist bereits bekannt. So wird in US 2003/0072842 A sowie von A. Bassoli et al., Quant. Struct.-Act. Relat., 20 (2001), S. 3-20, die Einzelverbindung 2-(3-Hydroxy-4-methoxyphenoxymethyl)benzoesäure offenbart. Diese Verbindung findet Verwendung als Süßungsmittel, ein Zusammenhang mit PPAR- Agonisten beziehungsweise Antagonisten ist jedoch nicht offenbart.

Bevorzugte Verbindungen der Formel (IV) weisen die folgende Definition auf:
X¹ ist Halogen, C₁-C₆-Alkyl, C₁-C₆-Alkoxy oder Trifluormethyl;
X² ist Halogen, C₁-C₆-Alkyl, C₁-C₆-Alkoxy oder Trifluormethyl;
m ist 0, 1, 2, 3 oder 4;
n ist 0, 1, 2, 3 oder 4;
   unter der Voraussetzung, dass X¹ = C₁-C₆-Alkoxy sich nicht in para- Position zum Ether - Fragment befindet.

Mehr bevorzugte Verbindungen der Formel (IV) weisen die folgende Definition auf:
X¹ ist Halogen, C₁-C₆-Alkyl, C₁-C₆-Alkoxy oder Trifluormethyl;
X² ist Halogen, C₁-C₆-Alkyl, C₁-C₆-Alkoxy oder Trifluormethyl;
m ist 0 oder 1;
n ist 0, 1oder 2;
   unter der Voraussetzung, dass X¹ = C₁-C₆-Alkoxy sich nicht in para- Position zum Ether - Fragment befindet.

Noch mehr bevorzugte Verbindungen der Formel (IV) weisen die folgende Definition auf:
X¹ ist Halogen, C₁-C₃-Alkyl oder Trifluormethyl;
X² ist Halogen, C₁-C₃-Alkyl, C₁-C₃-Alkoxy oder Trifluormethyl;
m ist 0 oder 1;
n ist 0, 1oder 2.

Besonders bevorzugte Verbindungen der Formel (IV) weisen die folgende Definition auf:
X¹ ist C₁-C₃-Alkyl oder Brom;
X² ist C₁-C₃-Alkyl, Fluor oder Chlor;
m ist 0 oder 1;
n ist 0, 1oder 2.

Ganz besonders bevorzugte Verbindungen der Formel (IV) weisen die folgende Definition auf:
X² ist C₁-C₃-Alkyl;
m ist 0;
n ist 0 oder 1.

Wie vorstehend beschrieben, können die Verbindungen der Formel (IV) nach Schritt a) des erfindungsgemäßen Verfahrens hergestellt werden. Sie eignen sich als Ausgangsverbindungen zur Synthese von PPAR-Agonisten beziehungsweise -Antagonisten der allgemeinen Formel (I) nach Schritt b) des erfindungsgemäßen Verfahrens.

Die nachfolgenden Beispiele sollen das Verfahren verdeutlichen, ohne es jedoch einzuschränken.

### 1. Herstellung von Verbindungen der Formel (IV)

### Beispiel 1.1: 2-(3-Hydroxy-phenoxyphethyl)-6-methyl-benzoesäure und deren Solvate mit N-Methylpyrrolidon

### 2-(3-Hydroxy-phenoxymethyl)-6-methyl-benzoesäure (Monosolvat mit N-Methylpyrrolidon)

7-Methylphthalid (30,1 g, 203 mmol), Resorcin (45,3 g, 407 mmol) und Kalium tert.-butylat (47,6 g, 424 mmol) werden in 300 ml N-Methylpyrrolidon (NMP) suspendiert und 21 h bei 140 °C gerührt. Man fügt nach dieser Zeit eine zweite Menge Kalium tert.-butylat (2,4 g, 21,4 mmol) hinzu und rührt weitere 2,5 h bei 140 °C. Man kühlt die Reaktionsmischung ab, gibt 800 ml Wasser hinzu und säuert bei 20 °C mit konzentrierter Salzsäure auf pH = 2 an. Nach zweistündigem Rühren bei 3 bis 5 °C folgt die Absaugung des hellbeigen Feststoffs, der mit Wasser gewaschen und danach getrocknet wird. Das isolierte Produkt enthält 2-(3-Hydrox-yphenoxymethyl)-6-methyl-benzoesäure und N-Methylpyrrolidon als Solvatmolekül in einem Verhältnis von 1:1. Ausbeute 77,7 %, Schmelzpunkt 98 bis 99 °C.

¹H NMR (400 MHz, DMSO-d6)δ

### a) 2-(3-Hydroxy-phenoxymethyl)-6-methyl-benzoesäure

13,3 (br s, 1H, COOH), 9,4 (br s 1H, OH), 7,34 (d, 1H) 7,33 (s, 1H), 7,25 (m, 1H), 7,04 (t, 1H), 6,40 - 6,37 (2H), 5,04 (s, 2H, CH₂), 2,69 (s, 3H, CH₃)

### b) N-Methylpyrrolidon

3,30 (t, 2H), 2,233 (s, 3H), 2,18 (t, 2H), 1,90 (m, 2H)

### 2-(3-Hydroxy-phenoxymethyl)-6-methyl-benzoesäure (Hemisolvat mit N-Methylpyrrolidon)

53,5 g (150 mmol) 2-(3-Hydroxy-phenoxymethyl)-6-methyl-benzoesäure * NMP warden in 250 ml Toluol unter Erwärmen gelöst und mit 10 K/h auf Raumtemperatur abgekühlt. Anschließend wird eine Stunde bei 3-5 °C nachgerührt. Das Kristallisat wird abgesaugt und mit Toluol gewaschen. Man erhält das Hemisolvat der 2-(3-Hydroxy-phenoxymethyl)-6-methyl-benzoesäure * 0,5 NMP in einer Ausbeute von 82 %; Schmelzpunkt 112 bis 113°C.

### 2-(3-Hydroxy-phenoxymethyl)-6-methyl-benzoesäure (Nonsolvat)

5,0 g (14 mmol) 2-(3-Hydroxy-phenoxymethyl)-6-methyl-benzoesäure * NMP werden in 50 ml Wasser zwei Stunden unter Rückfluss erhitzt. Nach Abkühlen wird der erhaltene Feststoff abgesaugt und mit Wasser gewaschen. Man erhält 2-(3-Hydroxyphenoxymethyl)-6-methyl-benzoesäure in einer Ausbeute von 86,4 %; Schmelzpunkt 154 bis 155 °C.

### Beispiel 1.2: 2-(3-Hydroxy-phenoxymethyl)-benzoesäure

Phthalid (15,0 g, 112 mmol), Resorcin (23,6 g, 214 mmol) und Kalium tert.-butylat (47,6 g, 220 mmol) werden in 150 ml N-Methylpyrrolidon (NMP) suspendiert und 5 h bei 140 °C gerührt. Man kühlt die Reaktionsmischung ab, gibt 400 ml Wasser hinzu und säuert bei 20 °C mit konzentrierter Salzsäure auf pH = 4 an. Die Lösung wird einmal mit 200 ml Ethylacetat und viermal mit 100 ml Ethylacetat ausgeschüttelt. Die vereinigten Ethylacetat-Phasen werden über Magnesiumsulfat getrocknet und zur Trockne eingeengt.

Man erhält ein hellbraunes Öl, das bei 0 °C kristallisiert. Die Kristalle enthalten ein Gemisch aus 2-(3-Hydroxy-phenoxymethyl)-benzoesäure und dem zweifach alkylierten Resorcin. Erhitzt man dieses Gemisch mit der 10-fachen Menge Ethylacetat, so geht die 2-(3-Hydroxy-phenoxymethyl)-benzoesäure in Lösung, das zweifach alkylierte Produkt bleibt als Feststoff zurück. Die Ethylacetatphase wird zur Trockne eingeengt, wobei 2-(3-Hydroxy-phenoxymethyl)-benzoesäure als hellbeige Kristalle zurückbleibt.

¹H NMR (400 MHz, DMSO-d6)δ

13,1 (br s, 1H, COOH), 9,4 (br s 1H, OH), 7,92 (q, 1H), 7,64 - 7,57 (2H), 7,43 (dt, 1H), 7,06 (m, 1H), 6,41 - 6,34 (3H), 5,34 (s, 2H, CH₂)

### b) Entsprechend überalkyliertes Produkt der Formel (IV)

13,1 (br s 2H, COOH), 7,94 (d, 2H), 7,66 - 7,58 (4H), 7,45 (d, 2H), 7,21 (t, 1H), 6,62 - 6,58 (2H), 5,44 (s, 4H, CH₂)

### Beispiel 1.3: 2-(3-Hydroxy-phenoxymethyl)-6-methyl-benzoesäure und deren Solvat mit N,N-Dimethylacetamid

### 2-(3-Hydroxy-phenoxymethyl)-6-methyl-benzoesäure (Solvat mit N,N-Dimethylacetamid im Verhältnis 5:2)

Die Durchführung erfolgt analog Beispiel 1.1, jedoch wird N,N-Dimethylacetamid (DMAA) anstelle von N-Methylpyrrolidon (NMP) eingesetzt. Das Produkt wird zur Reinigung in Toluol umkristallisiert. Sowohl Roh- als auch das umkristallisierte Produkt besitzen ein 2-(3-Hydroxy-phenoxymethyl)-6-methyl-benzoesäure : DMAA-Verhältnis von 5:2. Die Ausbeute beträgt 60,0 %, Schmelzpunkt 112 -114 °C.

### 2. Herstellung von Verbindungen der allgemeinen Formel (I)

### Beispiel 2.1: 2-{3-[2-(4-Fluor-phenyl)-oxazol-4-ylmethoxy]-phenoxymethyl}-6-methylbenzoesäure

### a) in N-Methylpyrrolidon als Lösungsmittel

15,9 g (51,6 mmol) 2-(3-Hydroxy-phenoxymethyl)-6-methyl-benzoesäure (Hemisolvat mit N-Methylpyrrolidon) werden in 70 ml N-Methylpyrrolidon unter Rühren bei Raumtemperatur mit 18,6 g 30%iger Lösung von Natriummethylat in Methanol (104 mmol) versetzt. Nach fünf Minuten gibt man 10,0 g (47,3 mmol) 4-Chlormethyl-2-(4-fluor-phenyl)oxazol hinzu, rührt 23 h bei Raumtemperatur und erwärmt abschließend eine Stunde auf 50 °C. Die Reaktionslösung wird mit 500 ml 19%iger Kochsalzlösung versetzt und die erhaltene Mischung zweimal mit 225 ml Ethylacetat ausgeschüttelt. Die vereinigten Ethylacetat-Phasen werden dreimal mit 225 ml Wasser ausgeschüttelt, die vereinigten Wasser-Phasen mit 2 M Salzsäure auf pH = 3 angesäuert. Die erhaltenen Kristalle werden abgesaugt und mit Wasser gewaschen. Die weitere Reinigung des Produkts erfolgt durch Kristallisation in Isopropanol. Man erhält 2-{3-[2-(4-Fluor-phenyl)-oxazol-4-ylmethoxy]-phenoxymethyl}-6-methyl-benzoesäure als farblose Kristalle in einer Ausbeute von 58,3 %, Schmelzpunkt 168 - 173 °C.

¹H NMR (400 MHz, DMSO-d6)δ

13,2 (br s, 1H, COOH), 8,29 (s, 1H, Oxazol-H), 8,04 (m, 2H, Fluorphenyl-H), 7,39 (m, 2H, Fluorphenyl-H), 7,35 - 7,31 (m, 2H), 7,25 (m, 1H), 7,20 (m, 1H), 6,67 - 6,65 (m, 2H), 6,58 (dd,1H), 5,11 (s, 2H, CH₂), 5,03 (s, 2H, CH₂), 2,33 (s, 3H, CH₃)

### b) in Methanol als Lösungsmittel

Die Reaktion wird wie unter a) beschrieben durchgeführt. Die Reaktionstemperatur beträgt über den gesamten Zeitraum von 24 Stunden 50 °C. Man erhält 2-{3-[2-(4-Fluor-phenyl)-oxazol-4-ylmethoxy]-phenoxymethyl}-6-methyl-benzoesäure als farblose Kristalle in einer Ausbeute von 53,0 %.

### Beispiel 2.2: 6-Methyl-2-[3-(2-phenyl-oxazol-4-ylmethoxy)-phenoxymethyl]-benzoesäure

Die Durchführung erfolgt analog Beispiel 2.1, jedoch wird 4-Chloromethyl-2-phenyloxazol anstelle von 4-Chlormethyl-2-(4-fluor-phenyl)-oxazol eingesetzt. Man erhält 2-{3-[2-phenyl-oxazol-4-ylmethoxy]-phenoxymethyl}-6-methyl-benzoesäure als farblose Kristalle in einer Ausbeute von 45,0 %.

¹H NMR (400 MHz, DMSO-d6)δ

13,2 (br s, 1H, COOH), 8,29 (s, 1H, Oxazol-H), 8,02 - 8,00 (m, 2H), 7,56 - 7,52 (m, 3H), 7,37 - 7,19 (4H), 6,69 - 6,59 (3H), 5,13 (s, 2H, CH₂), 5,05 (s, 2H, CH₂), 2,35 (s, 3H, CH₃)

### Beispiel 2.3: 2-(3-Benzyloxy-phenoxymethyl)-6-methyl-benzoesäure

Die Durchführung erfolgt analog Beispiel 2.1, jedoch wird Benzylbromid anstelle von 4-Chlormethyl-2-(4-fluor-phenyl)oxazol eingesetzt. Man erhält 2-(3-Benzyloxyphenoxymethyl)-6-methyl-benzoesäure als farblose Kristalle in einer Ausbeute von 47,3 %.

¹H NMR (400 MHz, DMSO-d6)δ

13,2 (br s, 1H, COOH), 7,45 - 7,30 (7H), 7,25 (m, 1H), 7,18 (t, 1H), 6,63 - 6,55 (3H), 5,10 (s, 2H, CH₂), 5,07 (s, 2H, CH₂), 5,34 (s, 3H, CH₃)

### Vergleichsbeispiel 2.4: (2-{3-[2-(4-Fluor-phenyl)-oxazol-4-ylmethoxy]-phenoxymethyl}-6-methyl-benzoesäure

1 g (6,8 mmol) 7-Methylphthalid und 2 g (7,0 mmol) 3-[2-(4-Fluoro-phenyl)-oxazol-4-ylmethoxy]phenol werden in NMP gelöst. Man fügt 3,9 ml methanolisches Natriummethylat (30%ig) hinzu, destilliert das Methanol ab und erwärmt auf 130 °C. Die Bildung von 2-{3-[2-(4-Fluor-phenyl)-oxazol-4-ylmethoxy]-phenoxymethyl}-6-methylbenzoesäure wird nicht beobachtet. Es kommt zur Zersetzung von 3-[2-(4-Fluorophenyl)oxazol-4-ylmethoxy]-phenol.

## Patentansprüche

1. Verfahren zur Herstellung einer Verbindung gemäß der allgemeinen Formel (I), **dadurch gekennzeichnet, dass**
a) eine Verbindung (II) in Gegenwart einer äquimolaren Menge einer Base B1 mit einer Verbindung (III) umgesetzt wird und 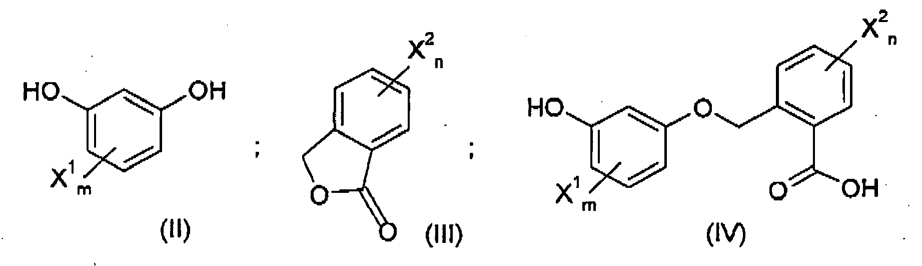
b) die in Schritt a) als Zwischenprodukt gebildete Verbindung (IV) in Gegenwart einer Base B2 mit einer Verbindung (V) zur Verbindung gemäß der allgemeinen Formel (I) umgesetzt wird, 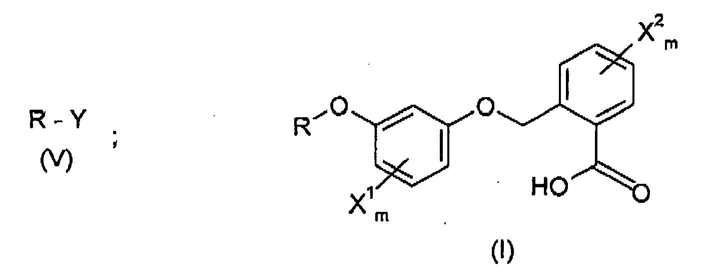
worin:
R ist ausgewählt aus der Gruppe bestehend aus:
unsubstituiertes oder zumindest monosubstituiertes C₁-C₁₀-Alkyl, Heterocyclyl, Aryl-(C₁-C₁₀-alkyl)-, Heteroaryl-(C₁-C₁₀-alkyl)- und Heterocyclyl-(C₁-C₁₀-alkyl)-,
wobei die Substituenten ausgewählt sind aus Halogen, C₁-C₆-Alkyl, -O-Aryl, Oxo, C₁-C₆-Alkoxy, -C(O)O-(C₁-C₆-Alkyl), C₂-C₆-Alkenyl, C₂-C₆-Alkinyl, -C(O)-(C₁-C₆-Alkyl), -C(O)NH₂, -C(O)NH(C₁-C₆-Alkyl), -C(O)N(C₁-C₆-Alkyl)₂, -S-(C₁-C₆-Alkyl), -SO₂NH₂, -SO₂-(C₁-C₆-Alkyl), -NH₂, -N(C₁-C₆-Alkyl)₂, -NH(C₁-C₆-Alkyl), -NO₂, -CN, Trifluormethyl, Trifluormethoxy, Aryl, Heterocyclyl und Heteroaryl,
und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆-Alkoxy, Trifluormethyl, Aryl, Heterocyclyl oder Heteroaryl,
wobei Aryl, Heterocyclyl und Heteroaryl wiederum mit C₁-C₃-Alkyl, C₁-C₃-Alkoxy, Halogen oder Trifluormethyl zumindest monosubstituiert sein können;
X² ist Halogen, C₁-C₆-Alkyl, C₁-C₆-Alkoxy, Trifluormethyl, Aryl, Heterocyclyl oder Heteroaryl,
wobei Aryl, Heterocyclyl und Heteroaryl wiederum mit C₁-C₃-Alkyl, C₁-C₃-Alkoxy, Halogen oder Trifluormethyl zumindest monosubstituiert sein können;
Heteroaryl ist ein 5- bis 14-gliedriger, aromatischer, mono-, bi- oder tricyclischer Heterocyclus, der ein oder mehrere Heteroatome ausgewählt aus N, O und S enthält;
Aryl ist ein 6- bis 14-gliedriger, aromatischer Mono-, Bi- oder Tricyclus;
Heterocyclyl ist ein 5- bis 14-gliedriger, nicht-aromatischer, mono-, bi- oder tricyclischer Heterocyclus, der ein oder mehrere Heteroatome ausgewählt aus N, O und S enthält;
m ist 0, 1, 2, 3 oder 4;
n ist 0, 1, 2, 3 oder 4;
Y ist eine Abgangsgruppe und
B2 ist ein Alkalihydroxid, Erdalkalihydroxid, Alkalialkoholat, Erdalkalialkoholat, Alkalihydrid, Erdalkalihydrid, Silazid, Alkaliamid oder Erdalkaliamid.

2. Verfahren gemäß Anspruch 1, **dadurch gekennzeichnet, dass** als Base B1 Natriummethylat, Kaliummethylat oder Kalium tert.-butylat verwendet wird.

3. Verfahren gemäß Anspruch 1' oder 2, **dadurch gekennzeichnet, dass** als Base B2 Natriummethylat, Kaliummethylat, Kalium tert.-butylat, Kalium-hexamethyldisilazid, Lithium-hexamethyldisilazid, Natriumhydrid, Calciumhydrid, Natriumhydroxid oder Kaliumhydroxid verwendet wird.

4. Verfahren gemäß einem der Ansprüche 1 bis 3, **dadurch gekennzeichnet, dass** in Schritt a) N-Methyl-1-pyrrolidon oder N,N-Dimethylacetamid als Lösungsmittel verwendet wird.

5. Verfahren gemäß einem der Ansprüche 1 bis 4, **dadurch gekennzeichnet, dass** in Schritt b) ein Alkohol oder ein Carbonsäureamid als Lösungsmittel verwendet wird.

6. Verfahren gemäß einem der Ansprüche 1 bis 5, **dadurch gekennzeichnet, dass** Schritt a) bei einer Temperatur zwischen 110 und 150°C und/oder Schritt b) bei einer Temperatur zwischen 20 und 50 °C durchgeführt wird.

7. Verfahren gemäß einem der Ansprüche 1 bis 6, **dadurch gekennzeichnet, dass**
in Schritt a) die Verbindung (II) in exakt äquimolarer Menge zur Base B1 und in mindestens äquimolarer Menge zur Verbindung (III) und/oder in Schritt b) die Base B2 in exakt 2-fach molarer Menge zur Verbindung (IV) und die Verbindung (V) in 1- bis 1,25-fach molarer Menge zur Verbindung (IV) eingesetzt werden.

8. Verfahren gemäß einem der Ansprüche 1 bis 7, **dadurch gekennzeichnet, dass** im Anschluss an Schritt b) aus einer Verbindung der Formel (I), die mindestens einen (-C(O)O-(C₁-C₆-Alkyl))-, (-S-(C₁-C₆-Alkyl))- oder (C₁-C₆-Alkoxy)-Substituenten aufweist, durch Alkylabspaltung eine Verbindung mit mindestens einem Carboxyl-, (-SH)- oder (-OH)-Substituenten hergestellt wird.

9. Verfahren gemäß einem der Ansprüche 1 bis 7, worin
R ausgewählt ist aus der Gruppe bestehend aus:
unsubstituiertes oder zumindest monosubstituiertes Aryl-(C₁-C₆-alkyl)- und Heteroaryl-(C₁-C₆-alkyl)-,
wobei die Substituenten ausgewählt sind aus Halogen, C₁-C₄-Alkyl, -O-Aryl, Oxo, C₁-C₄-Alkoxy, Aryl, Heterocyclyl und Heteroaryl,
und Aryl, Heterocyclyl und Heteroaryl wiederum mit C₁-C₃-Alkyl, C₁-C₃-Alkoxy, Halogen oder Trifluormethyl zumindest monosubstituiert sein können;
X¹ ist Halogen, C₁-C₆-Alkyl, C₁-C₆)-Alkoxy oder Trifluormethoxy;
X² ist Halogen, C₁-C₆-Alkyl, C₁-C₆-Alkoxy oder Trifluormethoxy
Heteroaryl ist ein 5- bis 14-gliedriger, aromatischer, mono-, bi- oder tricyclischer Heterocyclus, der ein oder mehrere Heteroatome ausgewählt aus N, O und S enthält;
Aryl ist ein 6- bis 14-gliedriger, aromatischer Mono-, Bi- oder Tricyclus;
Heterocyclyl ist ein 5- bis 14-gliedriger, nicht-aromatischer, mono-, bi- oder tricyclischer Heterocyclus, der ein oder mehrere Heteroatome ausgewählt aus N, O und S enthält;
m ist 0, 1 oder 2;
n ist 0, 1 oder 2;
Y ist eine Abgangsgruppe.

10. Verfahren gemäß Anspruch 9, worin:
R ist unsubstituiertes oder zumindest monosubstituiertes Benzyl oder Heteroarylmethyl, wobei die Substituenten ausgewählt sind aus Fluor, Chlor, C₁-C₄-Alkyl, -O-Phenyl, C₁-C₄-Alkoxy, Phenyl und Heteroaryl,
und Phenyl und Heteroaryl wiederum mit C₁-C₃-Alkyl, C₁-C₃-Alkoxy, Chlor oder Fluor zumindest monosubstituiert sein können;
X¹ ist C₁-C₃-Alkyl oder Brom;
X² ist C₁-C₃-Alkyl, Fluor oder Chlor;
Heteroaryl ist Oxazolyl, Isoxazolyl, Oxadiazolyl, Thiazolyl, Isothiazolyl, Pyridinyl, Pyridazinyl, pyrimidinyl, Pyrazinyl, Indolyl, Benzimidazolyl, Chinolinyl, Isochinolinyl, Chinazolinyl oder Chinoxalinyl;
m ist 0 oder 1;
n ist 0, 1 oder 2;
Y ist Chlor, Brom oder Iod.

## Claims

1. A process for the preparation of a compound of the general formula (I), wherein
a) a compound (II) is reacted in the presence of an equimolar amount of a base B1 with a compound (III) and 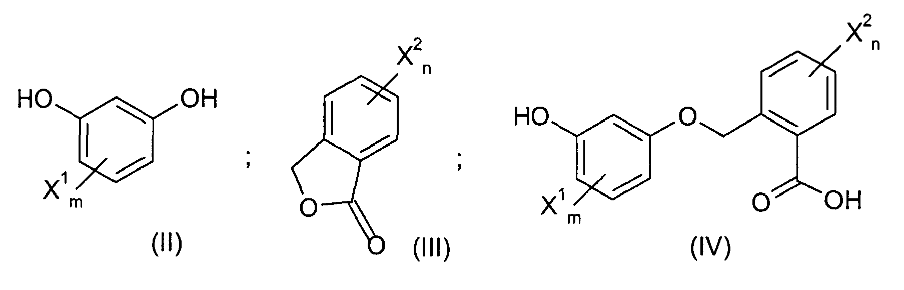
b) the compound (IV) formed as intermediate in step a) is reacted in the presence of a base B2 with a compound (V) to give the compound of the general formula (I) 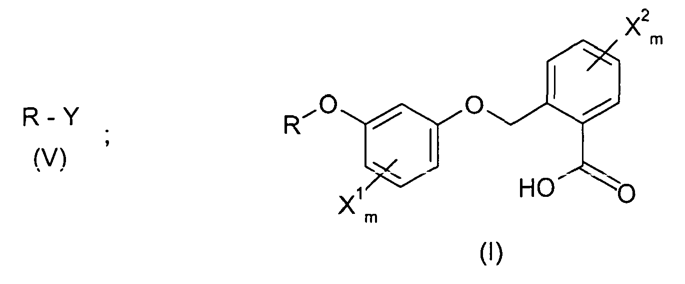
in which:
R is selected from the group consisting of:
unsubstituted or at least monosubstituted C₁-C₁₀-alkyl, heterocyclyl, aryl-(C₁-C₁₀-alkyl)-, heteroaryl-(C₁-C₁₀-alkyl)- and heterocyclyl-(C₁-C₁₀-alkyl)-,
where the substituents are selected from halogen, C₁-C₆-alkyl, -0-aryl, oxo, C₁-C₆-alkoxy, -C(O)O-(C₁-C₆-alkyl), C₂-C₆-alkenyl, C₂-C₆-alkynyl, -C(O)-(C₁-C₆-alkyl), -C(O)NH_{2,} -C(O)NH(C₁-C₆-alkyl), -C(O)N(C₁-C₆-alkyl)₂, -S-(C₁-C₆-alkyl), -SO₂NH₂, -SO₂-(C₁-C₆-alkyl), -NH₂, -N(C₁-C₆-alkyl)₂, -NH(C₁-C₆-alkyl), -NO₂, -CN, trifluoromethyl, trifluoromethoxy, aryl, heterocyclyl and heteroaryl,
and aryl, heterocyclyl and heteroaryl may in turn be at least monosubstituted by C₁-C₃-alkyl, C₁-C₃-alkoxy, halogen or trifluoromethyl;
X¹ is halogen, C₁-C₆-alkyl, C₁-C₆-alkoxy, trifluoromethyl, aryl, heterocyclyl or heteroaryl,
where aryl, heterocyclyl and heteroaryl may in turn be at least monosubstituted by C₁-C₃-alkyl, C₁-C₃-alkoxy, halogen or trifluoromethyl;
X² is halogen, C₁-C₆-alkyl, C₁-C₆-alkoxy, trifluoromethyl, aryl, heterocyclyl or heteroaryl,
where aryl, heterocyclyl and heteroaryl may in turn be at least monosubstituted by C₁-C₃-alkyl, C₁-C₃-alkoxy, halogen or trifluoromethyl;
heteroaryl is a 5- to 14-membered, aromatic, mono-, bi- or tricyclic heterocycle which contains one or more heteroatoms selected from N, 0 and S;
aryl is a 6- to 14-membered, aromatic mono-, bi- or tricyclic system;
heterocyclyl is a 5- to 14-membered, nonaromatic, mono-, bi- or tricyclic heterocycle which contains one or more heteroatoms selected from N, 0 and S;
m is 0, 1, 2, 3 or 4;
n is 0, 1, 2, 3 or 4;
Y is a leaving group and
B2 is an alkali metal hydroxide, alkaline earth metal hydroxide, alkali metal alcoholate, alkaline earth metal alcoholate, alkali metal hydride, alkaline earth metal hydride, silazide, alkali metal amide or alkaline earth metal amide.

2. The process as claimed in claim 1, wherein sodium methoxide, potassium methoxide or potassium tert-butoxide is used as base B1.

3. The process as claimed in claim 1 or 2, wherein sodium methoxide, potassium methoxide, potassium tert-butoxide, potassium hexamethyldisilazide, lithium hexamethyldisilazide, sodium hydride, calcium hydride, sodium hydroxide or potassium hydroxide is used as base B2.

4. The process as claimed in any of claims 1 to 3, wherein N-methyl-1-pyrrolidone or N,N-dimethylacetamide is used as solvent in step a).

5. The process as claimed in any of claims 1 to 4, wherein an alcohol or a carboxamide is used as solvent in step b).

6. The process as claimed in any of claims 1 to 5, wherein step a) is carried out at a temperature between 110 and 150°C and/or step b) is carried out at a temperature between 20 and 50°C.

7. The process as claimed in any of claims 1 to 6, wherein
in step a) the compound (II) is employed in exactly equimolar amount to the base B1 and in at least equimolar amount to the compound (III) and/or in step b) the base B2 is employed in exactly twice the molar amount to the compound (IV), and the compound (V) is employed in 1 to 1.25 times the molar amount to the compound (IV).

8. The process as claimed in any of claims 1 to 7, wherein step b) is followed by preparation of a compound having at least one carboxyl, (-SH) or (-OH) substituent by elimination of alkyl from a compound of the formula (I) which has at least one (-C(O)O-(C₁-C₆-alkyl)), (-S-(C₁-C₆-alkyl)) or (C₁-C₆-alkoxy) substituent.

9. The process as claimed in any of claims 1 to 7, in which
R is selected from the group consisting of:
unsubstituted or at least monosubstituted aryl-(C₁-C₆-alkyl)- and heteroaryl-(C₁-C₆-alkyl)-,
where the substituents are selected from halogen, C₁-C₄-alkyl, -O-aryl, oxo, C₁-C₄-alkoxy, aryl, heterocyclyl and heteroaryl,
and aryl, heterocyclyl and heteroaryl may in turn be at least monosubstituted by C₁-C₃-alkyl, C₁-C₃-alkoxy, halogen or trifluoromethyl;
X¹ is halogen, C₁-C₆-alkyl, C₁-C₆-alkoxy or trifluoromethoxy;
X² is halogen, C₁-C₆-alkyl, C₁-C₆-alkoxy or trifluoromethoxy;
heteroaryl is a 5- to 14-membered, aromatic, mono-, bi- or tricyclic heterocycle which contains one or more heteroatoms selected from N, O and S;
aryl is a 6- to 14-membered, aromatic mono-, bi- or tricyclic system;
heterocyclyl is a 5- to 14-membered, nonaromatic, mono-, bi- or tricyclic heterocycle which contains one or more heteroatoms selected from N, O and S;
m is 0, 1 or 2;
n is 0, 1 or 2;
Y is a leaving group.

10. The process as claimed in claim 9, in which
R is unsubstituted or at least monosubstituted benzyl or heteroarylmethyl, where the substituents are selected from fluorine, chlorine, C₁-C₄-alkyl, -O-phenyl, C₁-C₄-alkoxy, phenyl and heteroaryl,
and phenyl and heteroaryl may in turn be at least monosubstituted by C₁-C₃-alkyl, C₁-C₃-alkoxy, chlorine or fluorine;
X¹ is C₁-C₃-alkyl or bromine;
X² is C₁-C₃-alkyl, fluorine or chlorine;
heteroaryl is oxazolyl, isoxazolyl, oxadiazolyl, thiazolyl, isothiazolyl, pyridinyl, pyridazinyl, pyrimidinyl, pyrazinyl, indolyl, benzimidazolyl, quinolinyl, isoquinolinyl, quinazolinyl or quinoxalinyl;
m is 0 or 1;
n is 0, 1 or 2;
Y is chlorine, bromine or iodine.

## Revendications

1. Procédé de préparation d'un composé selon la formule générale (I), **caractérisé en ce que :**
a) un composé (II) est mis à réagir avec un composé (III), en présence d'une quantité équimolaire d'une base B1, et 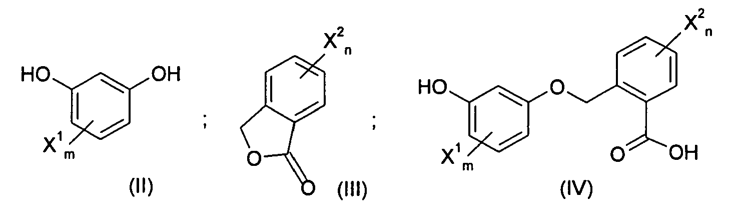
b) le composé (IV), formé en tant qu'intermédiaire dans l'étape a), est mis à réagir avec un composé (V), en présence d'une base B2, pour donner lieu au composé selon la formule générale (I) 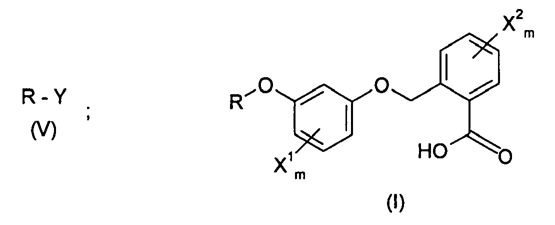
dans laquelle :
R est choisi parmi le groupe constitué :
d'un groupe alkyle en C₁-C₁₀, d'un groupe hétérocyclyle, d'un groupe aryl-(C₁-C₁₀-alkyl)-, d'un groupe hétéroaryl-(C₁-C₁₀-alkyl)- et d'un groupe hétérocyclyl-(C₁-C₁₀-alkyl)-, non substitué ou au moins mono-substitué,
où les substituants sont choisis parmi un atome d'halogène, un groupe alkyle en C₁-C₆, un groupe -O-aryle, un groupe oxo, un groupe alcoxy en C₁-C₆, un groupe -C(O)O-(C₁-C₆-alkyle), un groupe alcényle en C₂-C₆, un groupe alcynyle en C₂-C₆, un groupe -C(O)-(C₁-C₆-alkyle), un groupe -C(O)NH₂, un groupe -C(O)NH(C₁-C₆-alkyle), un groupe -C(O)N-(C₁-C₆-alkyle)₂, un groupe -S-(C₁-C₆-alkyle), un groupe -SO₂NH₂, un groupe -SO₂-(C₁-C₆-alkyle), un groupe -NH₂, un groupe -N(C₁-C₆-alkyle)₂, un groupe -NH(C₁-C₆-alkyle), un groupe -NO₂, un groupe -CN, un groupe trifluorométhyle, un groupe trifluoro-méthoxy, un groupe aryle, un groupe hétérocyclyle et un groupe hétéroaryle,
et un groupe aryle, un groupe hétérocyclyle et un groupe hétéroaryle peuvent, à nouveau, être au moins mono-substitués par un groupe alkyle en C₁-C₃, un groupe alcoxy en C₁-C₃, un atome d'halogène ou un groupe trifluorométhyle ;
X¹ est un atome d'halogène, un groupe alkyle en C₁-C₆, un groupe alcoxy en C₁-C₆, un groupe trifluorométhyle, un groupe aryle, un groupe hétérocyclyle ou un groupe hétéroaryle,
où un groupe aryle, un groupe hétérocyclyle et un groupe hétéroaryle peuvent, à nouveau, être au moins mono-substitués par un groupe alkyle en C₁-C₃, un groupe alcoxy en C₁-C₃, un atome d'halogène ou un groupe trifluorométhyle ;
X² est un atome d'halogène, un groupe alkyle en C₁-C₆, un groupe alcoxy en C₁-C₆, un groupe trifluorométhyle, un groupe aryle, un groupe hétérocyclyle ou un groupe hétéroaryle,
où un groupe aryle, un groupe hétérocyclyle et un groupe hétéroaryle peuvent, à nouveau, être au moins mono-substitués par un groupe alkyle en C₁-C₃, un groupe alcoxy en C₁-C₃, un atome d'halogène ou un groupe trifluorométhyle ;
un groupe hétéroaryle est un hétérocycle mono-, bi- ou tricyclique, aromatique, à 5-14 chaînons, qui renferme un ou plusieurs hétéroatomes choisis parmi un atome d'azote, un atome d'oxygène et un atome de soufre ;
un groupe aryle est un système mono-, bi- ou tricyclique, aromatique, à 6-14 chaînons ;
un groupe hétérocyclyle est un hétérocycle mono-, bi- ou tricyclique, non aromatique, à 5-14 chaînons, qui renferme un ou plusieurs hétéroatomes choisis parmi un atome d'azote, un atome d'oxygène et un atome de soufre ;
m vaut 0, 1, 2, 3 ou 4 ;
n vaut 0, 1, 2, 3 ou 4 ;
Y est un groupe partant, et
B2 est un hydroxyde de métal alcalin, un hydroxyde de métal alcalino-terreux, un alcoolate de métal alcalin, un alcoolate de métal alcalino-terreux, un hydrure de métal alcalin, un hydrure de métal alcalino-terreux, un silazide, un amidure de métal alcalin ou un amidure de métal alcalino-terreux.

2. Procédé selon la revendication 1, **caractérisé en ce que** l'on utilise le méthylate de sodium, le méthylate de potassium ou le tert-butylate de potassium en tant que base B1.

3. Procédé selon la revendication 1 ou 2, **caractérisé en ce que** l'on utilise le méthylate de sodium, le méthylate de potassium, le tert-butylate de potassium, l'hexaméthyldisilazide de potassium, l'hexaméthyldisilazide de lithium, l'hydrure de sodium, l'hydrure de calcium, l'hydroxyde de sodium ou l'hydroxyde de potassium en tant que base B2.

4. Procédé selon l'une quelconque des revendications 1 à 3, **caractérisé en ce que** l'on utilise de la N-méthyl-1-pyrrolidone ou du N,N-diméthylacétamide en tant que solvant dans l'étape a).

5. Procédé selon l'une quelconque des revendications 1 à 4, **caractérisé en ce que** l'on utilise un alcool ou un amide d'acide carboxylique en tant que solvant dans l'étape b).

6. Procédé selon l'une quelconque des revendications 1 à 5, **caractérisé en ce que** l'étape a) est réalisée à une température comprise entre 110 et 150°C et/ou l'étape b) est réalisée à une température comprise entre 20 et 50°C.

7. Procédé selon l'une quelconque des revendications 1 à 6, **caractérisé en ce que :**
dans l'étape a), on utilise le composé (II) en une quantité exactement équimolaire par rapport à la base B1 et en une quantité au moins équimolaire par rapport au composé (III) et/ou dans l'étape b), on utilise la base B2 en une quantité molaire supérieure d'un facteur 2 exactement par rapport au composé (IV) et le composé (V) en une quantité molaire supérieure d'un facteur 1 à 1,25 par rapport au composé (IV) .

8. Procédé selon l'une quelconque des revendications 1 à 7, **caractérisé en ce qu'**à la suite de l'étape b), un composé renfermant au moins un substituant carboxyle, (-SH) ou (-OH) est préparé par clivage de groupe alkyle à partir d'un composé de formule (I) qui renferme au moins un substituant (-C(O)O-(C₁-C₆-alkyle)), (-S-(C₁-C₆-alkyle)) ou alcoxy en C₁-C₆.

9. Procédé selon l'une quelconque des revendications 1 à 7, dans lequel :
R est choisi parmi le groupe constitué :
d'un groupe aryl-(C₁-C₆-alkyl)- et d'un groupe hétéroaryl-(C₁-C₆-alkyl)-, non substitué ou au moins mono-substitué,
où les substituants sont choisis parmi un atome d'halogène, un groupe alkyle en C₁-C₄, un groupe -O-aryle, un groupe oxo, un groupe alcoxy en C₁-C₄, un groupe aryle, un groupe hétérocyclyle et un groupe hétéroaryle,
et un groupe aryle, un groupe hétérocyclyle et un groupe hétéroaryle peuvent, à nouveau, être au moins mono-substitués par un groupe alkyle en C₁-C₃, un groupe alcoxy en C₁-C₃, un atome d'halogène ou un groupe trifluorométhyle ;
X¹ est un atome d'halogène, un groupe alkyle en C₁-C₆, un groupe alcoxy en C₁-C₆ ou un groupe tri-fluorométhoxy ;
X² est un atome d'halogène, un groupe alkyle en C₁-C₆, un groupe alcoxy en C₁-C₆ ou un groupe tri-fluorométhoxy ;
un groupe hétéroaryle est un hétérocycle mono, bi-
ou tricyclique, non aromatique, à 5-14 chaînons, qui renferme un ou plusieurs hétéroatomes choisis parmi un atome d'azote, un atome d'oxygène et un atome de soufre ;
un groupe aryle est un système mono-, bi- ou tricyclique, aromatique, à 6-14 chaînons ;
un groupe hétérocyclyle est un hétérocycle mono-, bi- ou tricyclique, non aromatique, à 5-14 chaînons, qui renferme un ou plusieurs hétéroatomes choisis parmi un atome d'azote, un atome d'oxygène et un atome de soufre ;
m vaut 0, 1 ou 2 ;
n vaut 0, 1 ou 2 ;
Y est un groupe partant.

10. Procédé selon la revendication 9, dans lequel :
R est un groupe benzyle ou un groupe hétéro-arylméthyle, non substitué ou au moins mono-substitué, où les substituants sont choisis parmi un atome de fluor, un atome de chlore, un groupe alkyle en C₁-C₄, un groupe -O-phényle, un groupe alcoxy en C₁-C₄, un groupe phényle et un groupe hétéroaryle,
et un groupe phényle et un groupe hétéroaryle peuvent, à nouveau, être au moins mono-substitués par un groupe alkyle en C₁-C₃, un groupe alcoxy en C₁-C₃, un atome de chlore ou un atome de fluor ;
X¹ est un groupe alkyle en C₁-C₃ ou un atome de brome ;
X² est un groupe alkyle en C₁-C₃, un atome de fluor
ou un atome de chlore ;
un groupe hétéroaryle est un groupe oxazolyle, un groupe isoxazolyle, un groupe oxadiazolyle, un groupe thiazolyle, un groupe isothiazolyle, un groupe pyridinyle, un groupe pyridazinyle, un groupe pyrimidinyle, un groupe pyrazinyle, un groupe indolyle, un groupe benzimidazolyle, un groupe quinoléinyle, un groupe isoquinoléinyle, un groupe quinazolinyle ou un groupe quinoxalinyle ;
m vaut 0 ou 1 ;
n vaut 0, 1 ou 2 ;
Y est un atome de chlore, un atome de brome ou un atome d'iode.
